# EUROPEAN PATENT SPECIFICATION

(11) **EP 3 651 116 B1**
(45) Date of publication and mention of the grant of the patent: **06.04.2022**
(21) Application number: 18205207.6
(22) Date of filing: 08.11.2018
(51) Int. Cl.: G06T 7/00, G06T 7/11, G06T 7/143

(54) **AUTONOMOUS SEGMENTATION OF THREE-DIMENSIONAL NERVOUS SYSTEM STRUCTURES FROM MEDICAL IMAGES**
AUTONOME SEGMENTIERUNG VON DREIDIMENSIONALEN NERVENSYSTEMSTRUKTUREN AUS MEDIZINISCHEN BILDERN
SEGMENTATION AUTONOME DE STRUCTURES TRIDIMENSIONNELLES DU SYSTÈME NERVEUX À PARTIR D'IMAGES MÉDICALES

(43) Date of publication of application: 13.05.2020
(73) Proprietor: Holo Surgical Inc., Chicago, IL 60609 (US)
(72) Inventor: Siemionow, Kris B., Chicago, IL 60610 (US); Luciano, Cristian J., Evergreen Park 60805 (US); Gawel, Dominik, 01-949 Warszawa (PL); Mejia Orozco, Edwing Isaac, 01-949 Warszawa (PL); Trzmiel, Michal, 01-949 Warszawa (PL)
(74) Representative: Kancelaria Eupatent.pl Sp. z.o.o

(56) References cited:
- HAN ZHONGYI ET AL: "Spine-GAN: Semantic segmentation of multiple spinal structures", MEDICAL IMAGE ANALYSIS, OXFORD UNIVERSITY PRESS, OXOFRD, GB, vol. 50, 25 August 2018 (2018-08-25), pages 23-35, XP085513247, ISSN: 1361-8415, DOI: 10.1016/J.MEDIA.2018.08.005
- Meelis Lootus ET AL: "Vertebrae Detection and Labelling in Lumbar MR Images", , 1 January 2014 (2014-01-01), XP055539506, Retrieved from the Internet: URL:https://www.robots.ox.ac.uk/~vgg/publi cations/2013/Lootus13/lootus13.pdf [retrieved on 2019-01-09]
- Charley Gros ET AL: "Automatic segmentation of the spinal cord and intramedullary multiple sclerosis lesions with convolutional neural networks", , 11 September 2018 (2018-09-11), XP055539507, United States Retrieved from the Internet: URL:https://arxiv.org/pdf/1805.06349 [retrieved on 2019-01-09]
- Patrick Ferdinand Christ ET AL: "Automatic Liver and Lesion Segmentation in CT Using Cascaded Fully Convolutional Neural Networks and 3D Conditional Random Fields", , 7 October 2016 (2016-10-07), XP055539508, Retrieved from the Internet: URL:https://arxiv.org/pdf/1610.02177 [retrieved on 2019-01-09]

## Description

### TECHNICAL FIELD

The present disclosure generally relates to autonomous segmentation of three-dimensional nervous system structures from medical images of human anatomy, which is useful in particular for the field of computer-assisted surgery, surgical navigation, surgical planning, and medical diagnostics.

### BACKGROUND

Image-guided or computer-assisted surgery is a surgical approach where the surgeon uses tracked surgical instruments in conjunction with preoperative or intraoperative images in order to indirectly guide the procedure. Image-guided surgery can utilize medical images acquired both preoperatively and intraoperatively, for example: from computed tomography (CT) or magnetic resonance imaging scanners.

Specialized computer systems can be used to process the medical images to develop three-dimensional (3D) models of the anatomy fragment subject to the surgery procedure. For this purpose, various machine learning technologies are being developed, such as a convolutional neural network (CNN) that is a class of deep, feed-forward artificial neural networks. CNNs use a variation of multilayer perceptrons designed to require minimal preprocessing.

A PCT patent application WO2016091833 (Arterys) discloses autonomous segmentation of anatomical structures, such as the human heart. The systems and methods employ convolutional neural networks (CNNs) to autonomously segment various parts of an anatomical structure represented by image data, such as 3D MRI data. The convolutional neural network utilizes two paths: a contracting path which includes convolution/pooling layers, and an expanding path which includes upsampling/convolution layers. The loss function used to validate the CNN model may specifically account for missing data, which allows for use of a larger training set. The CNN model may utilize multi-dimensional kernels (e.g., 2D, 3D, 4D, 6D), and may include various channels which encode spatial data, time data, flow data, etc. That system is designed specifically to process MRI images of soft tissue structures such as human heart. Moreover, it is not applicable to bony or nerve structure images, and in particular, to lower quality images that are difficult to segment.

A US patent application US2016328630 (Samsung) discloses an object recognition apparatus and method that can determine an image feature vector of a first image by applying a convolution network to the first image. The convolution network may extract features from image learning sets that include the first image and a sample segmentation map of the first image. The exemplary apparatus may determine a segmentation map of the first image by applying a deconvolution network to the determined image feature vector. The exemplary apparatus may determine a weight of the convolution network and a weight of the deconvolution network based on the sample segmentation map and the first segmentation map. The exemplary apparatus may determine a second segmentation map of a second image through the convolution network using the determined weight of the convolution network and through the deconvolution network using the determined weight of the deconvolution network.

In the field of image guided surgery, low quality images may make it difficult to adequately identify key anatomic landmarks, which may in turn lead to decreased accuracy and efficacy of the navigated tools and implants. Furthermore, low quality image datasets may be difficult to use in machine learning applications.

Computed tomography (CT) is a common method for generating a 3D volume of the anatomy. CT scanning works like other x-ray examinations. Very small, controlled amounts of x-ray radiation are passed through the body, and different tissues absorb radiation at different rates. With plain radiology, when special film is exposed to the absorbed x-rays, an image of the inside of the body is captured. With CT, the film is replaced by an array of detectors, which measure the x-ray profile.

The CT scanner contains a rotating gantry that has an x-ray tube mounted on one side and an arc-shaped detector mounted on the opposite side. An x-ray beam is emitted in a fan shape as the rotating frame spins the x-ray tube and detector around the patient. Each time the x-ray tube and detector make a 360° rotation and the x-ray passes through the patient's body, the image of a thin section is acquired. During each rotation, the detector records about 1,000 images (profiles) of the expanded x-ray beam. Each profile is then reconstructed by a dedicated computer into a 3D volume of the section that was scanned. The speed of gantry rotation, along with slice thickness, contributes to the accuracy/usefulness of the final image.

Commonly used intraoperative scanners have a variety of settings that allow for control of radiation dose. In certain scenarios high dose settings may be chosen to ensure adequate visualization of all the anatomical structures. The downside is increased radiation exposure to the patient. The effective doses from diagnostic CT procedures are typically estimated to be in the range of 1 to 10 mSv (millisieverts). This range is not much less than the lowest doses of 5 to 20 mSv estimated to have been received by survivors of the atomic bombs. These survivors, who are estimated to have experienced doses slightly larger than those encountered in CT, have demonstrated a small but increased radiation-related excess relative risk for cancer mortality.

The risk of developing cancer as a result of exposure to radiation depends on the part of the body exposed, the individual's age at exposure, the radiation dose, and the individual's gender. For the purpose of radiation protection, a conservative approach that is generally used is to assume that the risk for adverse health effects from cancer is proportional to the amount of radiation dose absorbed and that there is no amount of radiation that is completely without risk.

Low dose settings should be therefore selected for computed tomography scans whenever possible to minimize radiation exposure and associated risk of cancer development. However, low dose settings may have an impact on the quality of the final image available for the surgeon. This in turn can limit the value of the scan in diagnosis and treatment.

Magnetic resonance imaging (MRI) scanner forms a strong magnetic field around the area to be imaged. In most medical applications, protons (hydrogen atoms) in tissues containing water molecules create a signal that is processed to form an image of the body. First, energy from an oscillating magnetic field temporarily is applied to the patient at the appropriate resonance frequency. The excited hydrogen atoms emit a radio frequency signal, which is measured by a receiving coil. The radio signal may be made to encode position information by varying the main magnetic field using gradient coils. As these coils are rapidly switched on and off, they create the characteristic repetitive noise of an MRI scan. The contrast between different tissues is determined by the rate at which excited atoms return to the equilibrium state. Exogenous contrast agents may be given intravenously, orally, or intra-articularly.

The major components of an MRI scanner are: 1) the main magnet, which polarizes the sample, 2) the shim coils for correcting inhomogeneities in the main magnetic field, 3) the gradient system, which is used to localize the MR signal, and 4) the RF system, which excites the sample and detects the resulting NMR signal. The whole system is controlled by one or more computers.

The most common MRI strengths are 0.3T, 1.5T and 3T. The "T" stands for Tesla--the unit of measurement for the strength of the magnetic field. The higher the number, the stronger the magnet. The stronger the magnet the higher the image quality. For example, a 0.3T magnet strength will result in lower quality imaging then a 1.5T. Low quality images may pose a diagnostic challenge as it may be difficult to identify key anatomical structures or a pathologic process. Low quality images also make it difficult to use the data during computer assisted surgery. Thus, it is important to have the ability to deliver a high quality MR image for the physician.

A publication "Spine-GAN: Semantic segmentation of multiple spinal structures" by Han Zhongyi et al (Medical Image Analysis, Oxford University Press, Oxford, GB, vol. 50, 25 August 2018, pages 23-35) discloses automated segmentation and classification (i.e., normal and abnormal) of intervertebral discs, vertebrae, and neural foramen in MRIs.

Therefore, there is a need to develop a system and a method for efficiently segmenting three-dimensional nervous system structures from intraoperative and presurgical medical images in an autonomous manner, i.e. without human intervention in the segmentation process.

### SUMMARY

There is disclosed herein a method and system for autonomous segmentation of three-dimensional nervous system structures from raw medical images according to the appended claims.

### BRIEF DESCRIPTION OF DRAWINGS

The invention is shown by means of example embodiments on a drawing, wherein:
Fig. 1 shows a training procedure;
Figs. 2A-2C show exemplary images used in the system during the procedures;
Fig. 2D shows exemplary automatically defined region of interest used in the process;
Fig. 2E-1 and 2E-2 show three dimensional resizing of exemplary region of interest;
Fig. 2F shows exemplary transformation for data augmentation;
Fig. 3 shows an overview of a segmentation procedure;
Fig. 4 shows a general CNN architecture used for nervous system structure segmentation;
Fig. 5 shows a flowchart of a training process for the nervous system structure segmentation CNN;
Fig. 6 shows a flowchart of an inference process for the nervous system structures segmentation CNN;
Fig. 7 shows the result of the semantic segmentation of the spine parts and nervous system structures
Fig. 8 shows the model of the nervous system structures as a result from the segmentation CNN;
Fig. 9A shows the trajectory of a surgical implant colliding with a nervous system structure;
Fig. 9B shows the trajectory of a surgical instrument colliding with a nervous system structure;
Fig. 10 shows a computer-implemented system for implementing the segmentation procedure.

### DETAILED DESCRIPTION

The present invention relates to processing three dimensional images of nervous system structures in the vicinity of bones, such as nerves of extremities (arms and legs), cervical, thoracic or lumbar plexus, spinal cord (protected by the spinal column), nerves of the peripheral nervous system, cranial nerves, and others. The invention will be presented below based on an example of a spine as a bone in the vicinity of (and at least partially protecting) the nervous system structures, but the method and system can be equally well used for nervous system structures and other bones.

Moreover, the present invention may include, before segmentation, pre-processing of low quality images to improve their quality. This can be done by employing a method presented in a European patent application EP16195826 by the present applicant or any other pre-processing quality improvement method. The low quality images may be, for example, low dose computed tomography (LDCT) images or magnetic resonance images captured with a relatively low power scanner.

The foregoing description will present examples related to computed tomography (CT) images, but a skilled person will realize how to adapt the embodiments to be applicable to other image types, such as magnetic resonance images.

The nerve structure identification method as presented herein comprises two main procedures: 1) human-assisted (manual) training, and 2) computed autonomous segmentation.

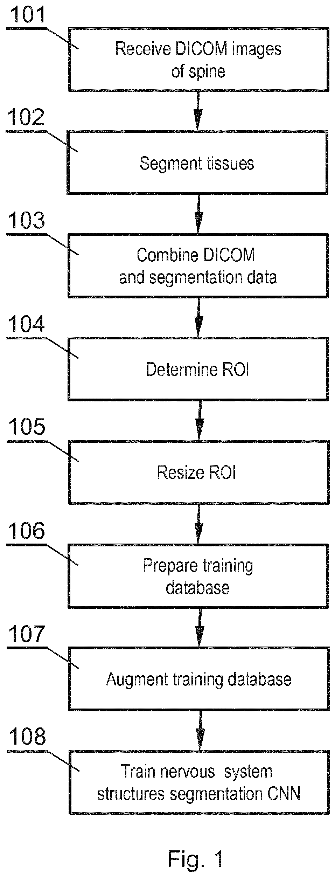

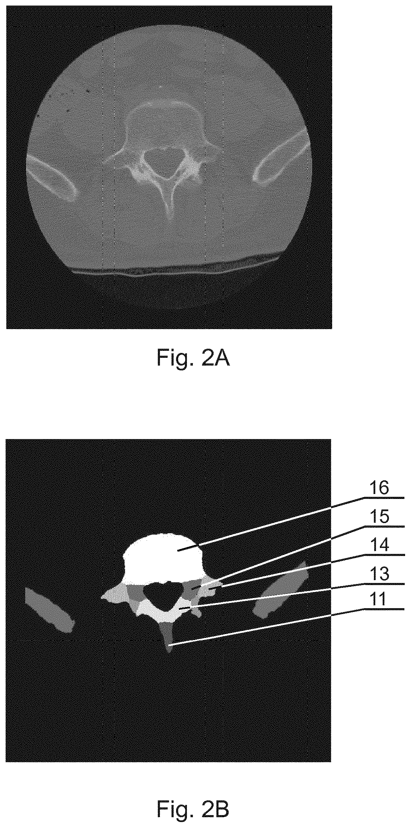

The training procedure, as presented in Fig. 1, comprises the following steps. First, in step 101, a set of DICOM (Digital Imaging and Communications in Medicine) images obtained with a preoperative or an intraoperative CT or MRI representing consecutive slices of the anatomy, with visible bony and soft tissues (such as one slice shown in Fig. 2A).

Next, the received images are processed in step 102 to perform autonomous segmentation of tissues, in order to determine separate areas corresponding to different parts of the bony structure, such as vertebral body 16, pedicles 15, transverse processes 14 and/or spinous process 11, as shown in Fig. 2B. For example, this can be done by employing a method for segmentation of images disclosed in a European patent application EP16195826 by the present applicant, or any other segmentation method.

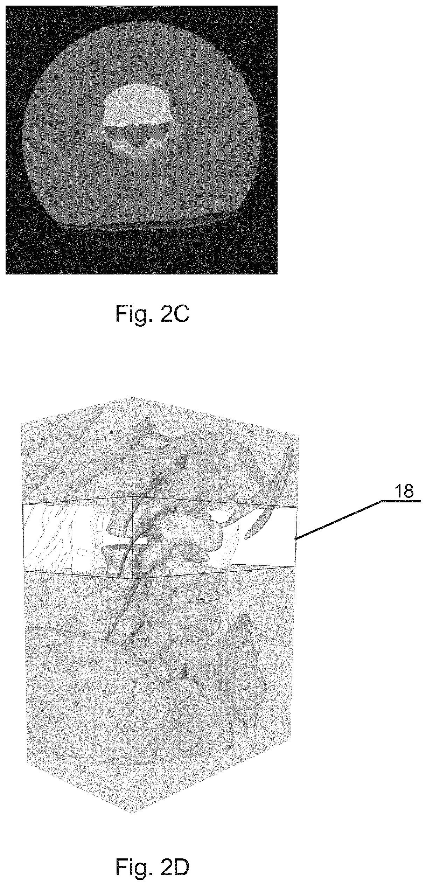

Then, in step 103, the information obtained from both original DICOM images and segmentation results is merged to obtain combined image, comprising information about the tissue appearance and its classification (including assignment of structure parts to classes corresponding to different anatomy parts), for example in a form of a color-coded DICOM image, as shown in Fig. 2C. Alternatively, separate DICOM (Fig. 2A) and segmentation (Fig. 2B) images can be processed instead of the combined image.

Next, in step 104, from the set of slice images a 3D region of interest (ROI) 18 is determined, that contains, for example, a volume of each vertebral level with a part of surrounding tissues including the nervous system structures and other structures such as muscles, vessels, ligaments, intervertebral discs, joints, cerebrospinal fluid, and others, as shown in Fig. 2D.

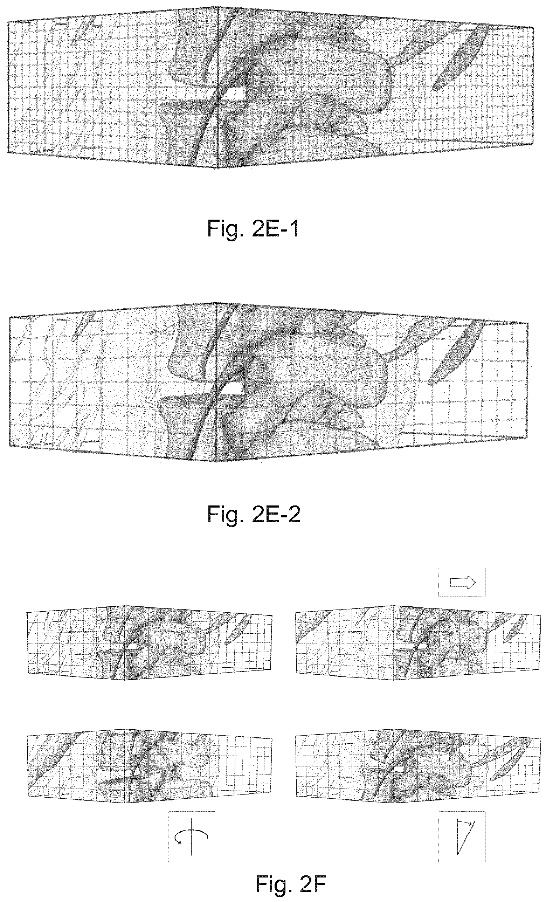

Then, in step 105, the 3D resizing of the determined ROI 18 is performed to achieve the same size of all ROI's stacked in the 3D matrices, each containing information about voxel distribution along X, Y and Z axes and the appearance and classification information data of bony structure, such as shown in Fig. 2E. In other words, the voxels are small cuboidal volumes resembling points having 3D coordinates and both the original radiodensity value obtained by the scanner and the assigned bony structure classification obtained by the segmentation algorithm.

Next, in step 106, a training database is prepared by a human, that comprises the previously determined ROIs and corresponding manually segmented nervous system structures.

Next, in step 107, the training database is augmented, for example with the use of a 3D generic geometrical transformation and resizing with dense 3D grid deformations, as shown in Fig. 2F. Data augmentation is performed on the images to make the training set more diverse. The foregoing transformations are remapping the voxels positions in a 3D ROI 18 based on a randomly warped artificial grid assigned to the ROI 18 volume. A new set of voxel positions is calculated artificially warping the 3D tissue shape and appearance. Simultaneously, the information about the tissue classification is warped to match the new tissue shape and the manually determined nervous system structure is recalculated in the same manner. During the process, the value of each voxel, containing information about the tissue appearance, is recalculated in regards to its new position in ROI 18 with use of an interpolation algorithm (for example bicubic, polynomial, spline, nearest neighbor, or any other interpolation algorithm) over the 3D voxel neighborhood.

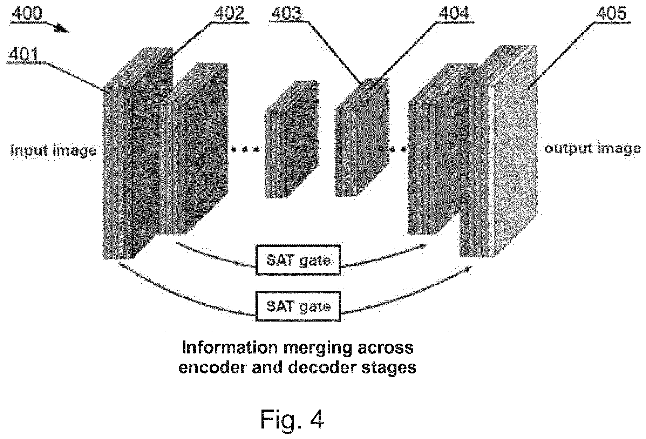

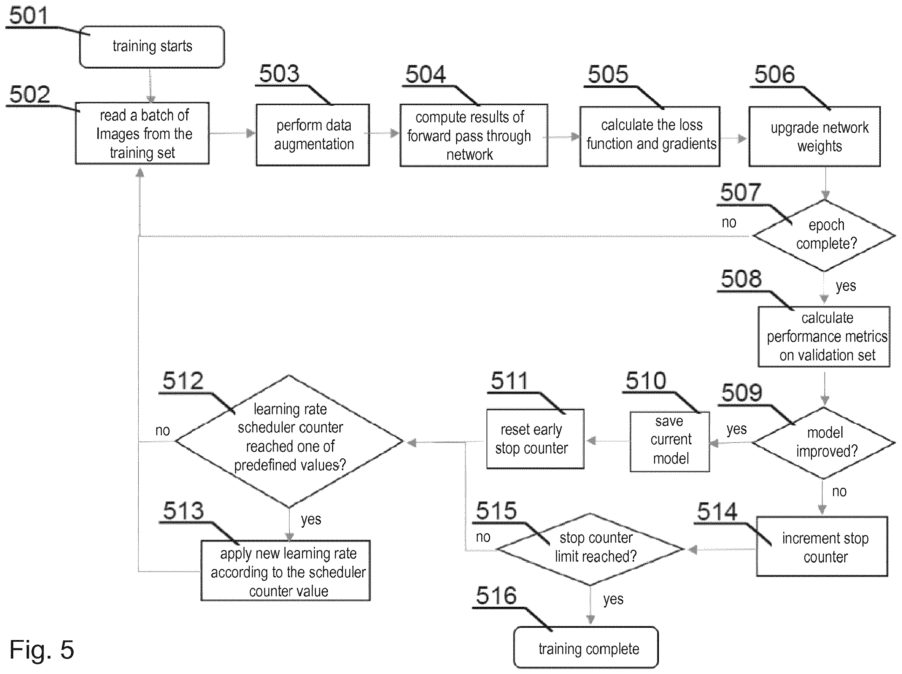

Then, in step 108, a convolutional neural network (CNN) is trained with manually segmented images (by a human) to segment the nervous system structures. Preferably, a network with a plurality of layers can be used, specifically a combination of convolutional with ReLU activation functions or any other non-linear or linear activation functions. For example, a network such as shown in Fig. 4 can be trained according to a process such as shown in Fig. 5. Additionally Select-Attend-Transfer (SAT) gates or Generative Adversarial Networks (GAN) can be used to increase the final quality of the segmentation.

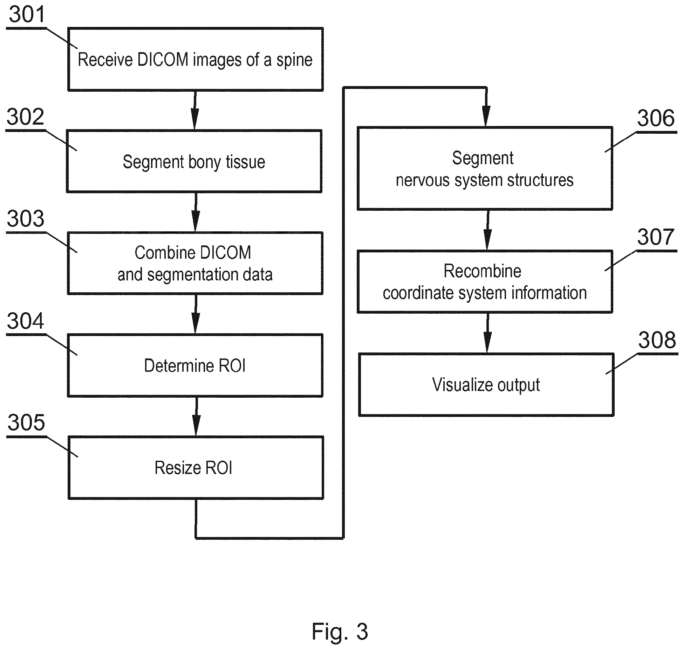

The segmentation procedure, as presented in Fig. 3, comprises the following steps. First, in step 301, a 3D scan volume is received, comprising a set of DICOM images of a region of the spinal anatomy. The 3D scan volume can be obtained from a preoperative or an intraoperative CT or MRI. The set of DICOMs representing consecutive slices of the anatomy is received (such as one slice shown in Fig. 2A). Next, the received images are processed in step 302 to perform autonomous segmentation of bony tissues to obtain bony structure segmentation data,, such as to determine separate areas corresponding to different spine parts, for example: vertebral body 16, pedicles 15, transverse processes 14, lamina 13 and/or spinous process 11, as shown in Fig. 2B. This step can be done by employing a method for segmentation of images disclosed in a European patent application EP16195826 by the present applicant or any other segmentation method. Then, in step 303, the information obtained from DICOM images and the bony structure segmentation data are merged to obtain combined image comprising information about the tissue appearance and its classification, for example in a form of a color-coded DICOM image, as shown in Fig. 2C. Alternatively, separate DICOM (Fig. 2A) and segmentation (Fig. 2B) images can be processed instead of the combined image. Next, in step 304, a 3D region of interest (ROI) 18 is autonomously determined, which contains a 3D subvolume of the bony structure with a part of surrounding tissues including the nervous system structure and other anatomical components, such as muscles, vessels, ligaments, intervertebral discs, joints, cerebrospinal fluid, and others, as shown in Fig. 2D. Then, in step 305, the 3D resizing of the determined ROI 18 is performed to achieve the same size of all ROI's stacked in the 3D matrices. Each 3D matrix contains information about voxel distribution along X, Y and Z axes with bone density and classification information data for bony structure, such as shown in Fig. 2E. Therefore, steps 301-305 are performed in a way similar to steps 101-105 of the training procedure of Fig. 1.

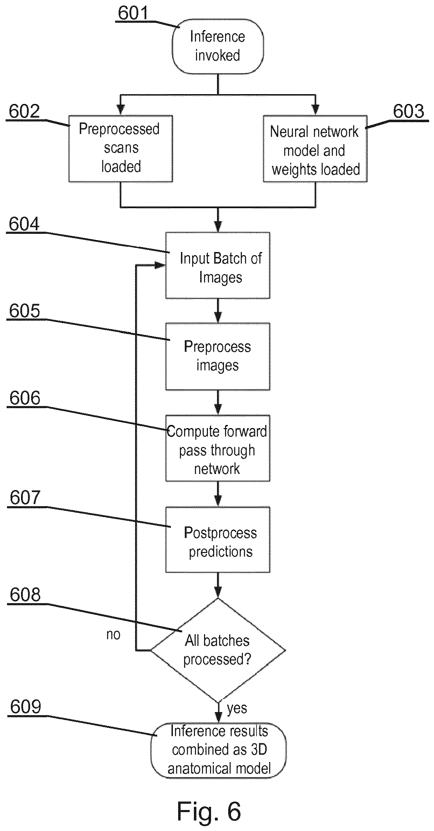

Next, in step 306, the nervous system structures are autonomously segmented by processing the resized ROI to determine the 3D size and shape of the nervous system structure, by means of the pretrained nervous-system-structure segmentation CNN 400, as shown in Fig. 4, according to the segmentation process presented in Fig. 6.

In step 307 the information about the global coordinate system (ROI position in the DICOM dataset) and local ROI coordinate system (segmented nervous system structures size, shape and position inside the ROI) is recombined.

Next, in step 308, the output, including the segmented nervous system structures, is visualized.

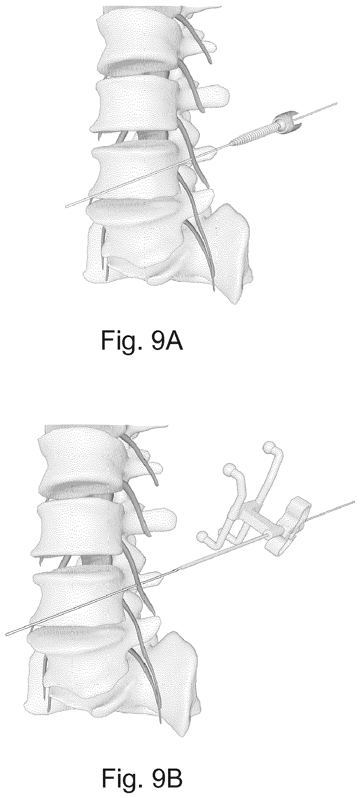

Anatomical knowledge of position, size, and shape of nervous system structures allow for real-time calculation of a possible collision detection with nervous system structures (Fig. 9A and 9B) while placing medical devices, for example while using a surgical navigation method presented in a European patent application EP18188557.5 by the present applicant. Such collision may results in nervous system structure damage, affecting patient health and quality of life. Autonomous real-time comparison of the position, size, and shape of the nervous system structures, and the medical devices upcoming position, with regard to their size and shape, allow for presenting warnings in the graphical user interface, for example such as presented in a European patent application EP18188557.5 by the present applicant. Moreover, the autonomous collision analysis allows for calculation of change of the prefered medical device position, and can be incorporated, for example, in the method presented in a European patent application EP18188557.5 by the present applicant.

Fig. 4 shows a convolutional neural network (CNN) architecture 400, hereinafter called the nervous-system-structure segmentation CNN, which is utilized in the present method for both semantic and binary segmentation. The network performs pixel-wise class assignment using an encoder-decoder architecture, using at least one input as a 3D information about the appearance (radiodensity) and the classification of bony structure in a 3D ROI. The left side of the network is a contracting path, which includes convolution layers 401 and pooling layers 402, and the right side is an expanding path, which includes upsampling or transpose convolution layers 403 and convolutional layers 404 and the output layer 405.

One or more 3D ROI's can be presented to the input layer of the network to learn reasoning from the data.

The type of convolution layers 401 can be standard, dilated, or hybrid s thereof, with ReLU, leaky ReLU or any other kind of activation function attached.

The type of upsampling or deconvolution layers 403 can also be standard, dilated, or hybrid thereof, with ReLU or leaky ReLU activation function attached.

The output layer 405 denotes the densely connected layer with one or more hidden layer and a softmax or sigmoid stage connected as the output.

The encoding-decoding flow is supplemented with additional skipping connections of layers with corresponding sizes (resolutions), which improves performance through information merging. It enables either the use of max-pooling indices from the corresponding encoder stage to downsample, or learning the deconvolution filters to upsample.

The general CNN architecture can be adapted to consider ROI's of different sizes. The number of layers and number of filters within a layer are also subject to change depending on the anatomical areas to be segmented.

The final layer for binary segmentation recognizes two classes: 1) nervous system structure, and 2) the background).

Additionally Select-Attend-Transfer (SAT) gates or Generative Adversarial Networks (GAN) can be used to increase the final quality of the segmentation. Introducing Select-Attend-Transfer gates to the encoder-decoder neural network results in focusing the network on the most important tissue features and their localization, simultaneously decreasing the memory consumption. Moreover, the Generative Adversarial Networks can be used to produce new artificial training examples.

The semantic segmentation is capable of recognizing multiple classes, each representing a part of the anatomy. For example the nervous system structure may include nerves of the upper and lower extremities, cervical, thoracic or lumbar plexus, the spinal cord, nerves of the peripheral nervous system (e.g., sciatic nerve, median nerve, brachial plexus), cranial nerves, and others.

Fig. 5 shows a flowchart of a training process, which can be used to train the nervous-system-structure segmentation CNN 400. The objective of the training for the segmentation CNN 400 is to tune the parameters of the segmentation CNN 400, so that the network is able to recognize and segment a 3D image (ROI). The training database may be split into a training set used to train the model, a validation set used to quantify the quality of the model, and a test set.

The training starts at 501. At 502, batches of training 3D images (ROIs) are read from the training set, one batch at a time. For the segmentation, 3D images (ROIs) represent the input of the CNN, and the corresponding pre-segmented 3D images (ROIs), which were manually segmented by a human, represent its desired output.

At 503 the original 3D images (ROIs) can be augmented. Data augmentation is performed on these 3D images (ROIs) to make the training set more diverse. The input and output pair of three dimensional images (ROIs) is subjected to the same combination of transformations.

At 504, the original 3D images (ROIs) and the augmented 3D images (ROIs) are then passed through the layers of the CNN in a standard forward pass. The forward pass returns the results, which are then used to calculate at 505 the value of the loss function (i.e., the difference between the desired output and the output computed by the CNN). The difference can be expressed using a similarity metric (e.g., mean squared error, mean average error, categorical cross-entropy, or another metric).

At 506, weights are updated as per the specified optimizer and optimizer learning rate. The loss may be calculated using a per-pixel cross-entropy loss function and the Adam update rule.

The loss is also back-propagated through the network, and the gradients are computed. Based on the gradient values, the network weights are updated. The process, beginning with the 3D images (ROIs) batch read, is repeated continuously until an end of the training session is reached at 506.

Then, at 508, the performance metrics are calculated using a validation dataset - which is not explicitly used in training set. This is done in order to check at 509 whether not the model has improved. If it is not the case, the early stop counter is incremented by one at 514, as long as its value has not reached a predefined maximum number of epochs at 515. The training process continues until there is no further improvement obtained at 516. Then the model is saved at 510 for further use, and the early stop counter is reset at 511. As the final step in a session, learning rate scheduling can be applied. The session at which the rate is to be changed are predefined. Once one of the session numbers is reached at 512, the learning rate is set to one associated with this specific session number at 513.

Once the training process is complete, the network can be used for inference (i.e., utilizing a trained model for autonomous segmentation of new medical images).

Fig. 6 shows a flowchart of an inference process for the nervous-system-structure segmentation CNN 400.

After inference is invoked at 601, a set of scans (three dimensional images) are loaded at 602 and the segmentation CNN 400 and its weights are loaded at 603.

At 604, one batch of three dimensional images (ROIs) at a time is processed by the inference server.

At 605, the images are preprocessed (e.g., normalized, cropped, etc.) using the same parameters that were utilized during training. In at least some implementations, inference-time distortions are applied and the average inference result is taken on, for example, 10 distorted copies of each input 3D image (ROI). This feature creates inference results that are robust to small variations in brightness, contrast, orientation, etc.

At 606, a forward pass through the segmentation CNN 400 is computed.

At 606, the system may perform post-processing such as linear filtering (e.g., Gaussian filtering), or nonlinear filtering ( e.g., median filtering, and morphological opening or closing).

At 608, if not all batches have been processed, a new batch is added to the processing pipeline until inference has been performed at all input 3D images (ROIs).

Finally, at 609, the inference results are saved and can be combined to a segmented 3D anatomical model. The model can be further converted to a polygonal mesh for the purpose of visualization. The volume and/or mesh representation parameters can be adjusted in terms of change of color, opacity, changing the mesh decimation depending on the needs of the operator.

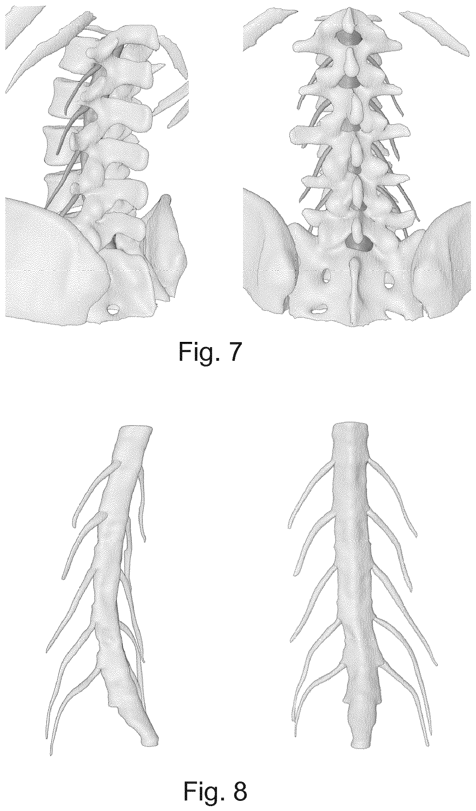

Fig. 7 shows a sample 3D model, derived from autonomous segmentation, converted to a polygonal mesh.

Fig. 8 shows a sample 3D model, derived from autonomously segmented images presenting a nervous system structure alone.

Fig. 9 shows a sample of the trajectory of a surgical implant (Fig. 9A) and an instrument (Fig. 9B) that collide with the segmented nervous system structure.

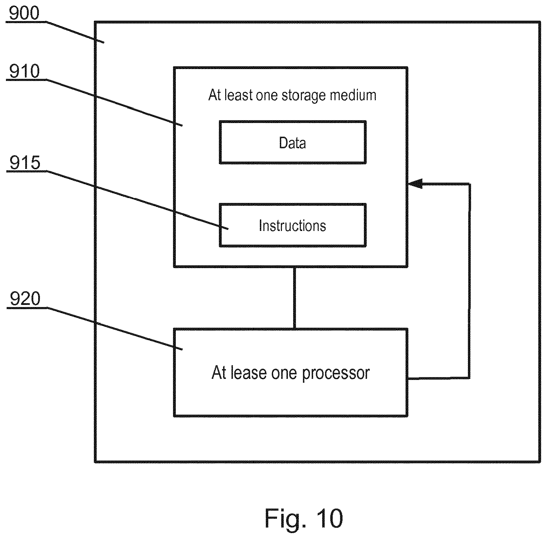

The functionality described herein can be implemented in a computer-implemented system 900, such as shown in Fig. 10. The system may include at least one non-transitory processor-readable storage medium that stores at least one of processor-executable instructions or data and at least one processor communicably coupled to at least one non-transitory processor-readable storage medium. At least one processor is configured to perform the steps of the methods presented herein.

The computer-implemented system 900, for example a machine-learning system, may include at least one non-transitory processor-readable storage medium 910 that stores at least one of processor-executable instructions 915 or data; and at least one processor 920 communicably coupled to the at least one non-transitory processor-readable storage medium 910. At least one processor 920 may be configured to (by executing the instructions 915) to perform the steps of the method of Fig. 3.

## Claims

1. A method for autonomous segmentation of three-dimensional nervous system structures from raw medical images, the method comprising the following steps:
- receiving (301) a 3D scan volume comprising a set of medical scan images of a region of the anatomy;
- autonomously processing (302) the set of medical scan images to perform segmentation of a bony structure of the anatomy to obtain bony structure segmentation data; **characterized by** further comprising the steps of:
- autonomously processing (304) a subsection of the 3D scan volume as a 3D region of interest ROI, by combining (303) the raw medical scan images and the bony structure segmentation data, wherein the 3D ROI contains a subvolume of the bony structure with a portion of surrounding tissues, including the nervous system structure, wherein the 3D ROI includes 3D matrices, each 3D matrix containing information about voxel distribution along X, Y and Z axes with bone density obtained from the 3D scan volume and classification information data for bony structure;
- autonomously processing the ROI (306) to determine the 3D shape, location, and size of the nervous system structures by means of a pre-trained convolutional neural network, CNN, (400).

2. The method according to claim 1, further comprising 3D resizing (305) of the ROI.

3. The method according to any of previous claims, further comprising visualizing (308) the output including the segmented nervous system structures.

4. The method according to any of the previous claims, further comprising detecting collision between an embodiment and/or trajectory of surgical instruments or implants and the segmented nervous system structures.

5. The method according to any of previous claims, wherein the nervous-system-structure segmentation CNN (400) is a fully convolutional neural network model with layer skip connections.

6. The method according to claim 5, wherein the nervous-system-structures segmentation CNN (400) output is improved by Select-Attend-Transfer, SAT, gates.

7. The method according to claim 5, wherein the nervous-system-structures segmentation CNN (400) output is improved by Generative Adversarial Networks, GAN.

8. The method according to any of previous claims, wherein the received medical scan images are collected from an intraoperative scanner.

9. The method according to any of previous claims, wherein the received medical scan images are collected from a presurgical stationary scanner.

10. A computer-implemented system, comprising:
- at least one non-transitory processor-readable storage medium (910) that stores at least one processor-executable instruction (915) or data; and
- at least one processor (920) communicably coupled to at least one non-transitory processor-readable storage medium (910), wherein at least one processor (920) is configured to perform the steps of the method of any of previous claims.

## Patentansprüche

1. Verfahren zur autonomen Segmentierung von dreidimensionalen Nervensystemstrukturen aus medizinischen Rohbildern, wobei das Verfahren die folgenden Schritte umfasst:
- Empfangen (301) eines 3D-Abtastvolumens, das einen Satz von medizinischen Abtastbildern eines Bereichs der Anatomie umfasst;
- autonomes Verarbeiten (302) des Satzes von medizinischen Abtastbildern, um Segmentierung einer knöchernen Struktur der Anatomie durchzuführen, um Segmentierungsdaten der knöchernen Struktur zu erhalten;
**dadurch gekennzeichnet, dass** es ferner die folgenden Schritte umfasst:
- autonomes Verarbeiten (304) eines Teilabschnittes des 3D-Abtastvolumens als einen 3D-Bereich von Interesse, ROI, durch Kombinieren (303) der medizinischen Rohabtastbilder und der Segmentierungsdaten der knöchernen Struktur, wobei der 3D-ROI ein Teilvolumen der knöchernen Struktur mit einem Teil aus umliegendem Gewebe enthält, beinhaltend die Nervensystemstruktur, wobei der 3D-ROI 3D-Matrizen beinhaltet, wobei jede 3D-Matrix Informationen über Voxelverteilung entlang X-, Y- und Z-Achsen mit Knochendichte erhalten aus dem 3D-Abtastvolumen und Klassifizierungsinformationsdaten für knöcherne Struktur enthält;
- autonomes Verarbeiten des ROI (306), um die 3D-Form, Stelle und Größe der Nervensystemstrukturen mittels eines vortrainierten neuronalen Faltungsnetzwerks, CNN, (400) zu bestimmen.

2. Verfahren nach Anspruch 1, ferner umfassend das 3D-Größenändern (305) des ROI.

3. Verfahren nach einem der vorhergehenden Ansprüche, ferner umfassend das Visualisieren (308) der Ausgabe, welche die segmentierten Nervensystemstrukturen beinhaltet.

4. Verfahren nach einem der vorhergehenden Ansprüche, ferner umfassend das Erfassen von Kollision zwischen einer Ausführungsform und/oder Trajektorie von chirurgischen Instrumenten oder Implantaten und den segmentierten Nervensystemstrukturen.

5. Verfahren nach einem der vorhergehenden Ansprüche, wobei das CNN (400) der Nervensystemstruktursegmentierung ein Modell eines vollständigen neuronalen Faltungsnetzwerks mit Ebenensprungverbindungen ist.

6. Verfahren nach Anspruch 5, wobei die Ausgabe des CNN (400) der Nervensystemstruktursegmentierung durch Select-Attend-Transfer-, SAT-, Gates verbessert wird.

7. Verfahren nach Anspruch 5, wobei die Ausgabe des CNN (400) der Nervensystemstruktursegmentierung durch Generative Adversarial Networks, GAN, verbessert wird.

8. Verfahren nach einem der vorhergehenden Ansprüche, wobei die empfangenen medizinischen Abtastbilder von einem intraoperativen Abtaster gesammelt werden.

9. Verfahren nach einem der vorhergehenden Ansprüche, wobei die empfangenen medizinischen Abtastbilder von einem vorchirurgischen stationären Abtaster gesammelt werden.

10. Computerimplementiertes System, umfassend:
- zumindest ein nichtflüchtiges prozessorlesbares Speichermedium (910), das zumindest eine prozessorausführbare Anweisung (915) oder Daten speichert; und
- zumindest einen Prozessor (920), der kommunizierbar an zumindest ein nichtflüchtiges prozessorlesbares Speichermedium (910) gekoppelt ist, wobei zumindest ein Prozessor (920) konfiguriert ist, um die Schritte des Verfahrens nach einem der vorhergehenden Ansprüche durchzuführen.

## Revendications

1. Procédé de segmentation autonome de structures tridimensionnelles du système nerveux à partir d'images médicales brutes, le procédé comprenant les étapes suivantes :
- la réception (301) d'un volume de balayage 3D comprenant un ensemble d'images de balayage médical d'une région de l'anatomie ;
- le traitement autonome (302) de l'ensemble d'images de balayage médical pour effectuer la segmentation d'une structure osseuse de l'anatomie afin d'obtenir des données de segmentation de structure osseuse ;
**caractérisé en ce qu'**il comprend en outre les étapes de :
- traitement autonome (304) d'une sous-section du volume de balayage 3D en tant que région d'intérêt, ROI, 3D par combinaison (303) des images de balayage médical brutes et des données de segmentation de structure osseuse, ladite ROI 3D contenant un sous-volume de la structure osseuse avec une partie des tissus environnants, y compris la structure du système nerveux, ladite ROI 3D comprenant des matrices 3D, chaque matrice 3D contenant des informations sur la distribution des voxels le long des axes X, Y et Z avec la densité osseuse obtenue à partir du volume de balayage 3D et des données d'informations de classification pour la structure osseuse ;
- traitement autonome de la ROI (306) pour déterminer la forme, l'emplacement et la taille en 3D des structures du système nerveux au moyen d'un réseau neuronal convolutionnel, CNN, pré-entraîné (400).

2. Procédé selon la revendication 1, comprenant en outre un redimensionnement 3D (305) de la ROI.

3. Procédé selon l'une quelconque des revendications précédentes, comprenant en outre la visualisation (308) du résultat comprenant les structures segmentées du système nerveux.

4. Procédé selon l'une quelconque des revendications précédentes, comprenant en outre la détection de la collision entre un mode de réalisation et/ou une trajectoire d'instruments chirurgicaux ou d'implants et les structures segmentées du système nerveux.

5. Procédé selon l'une quelconque des revendications précédentes, ledit CNN de segmentation (400) de la structure du système nerveux étant un modèle de réseau neuronal entièrement convolutionnel avec connexions par saut de couche.

6. Procédé selon la revendication 5, ledit résultat du CNN de segmentation (400) des structures du système nerveux étant amélioré par des portes Select-Attend-Transfer, SAT.

7. Procédé selon la revendication 5, ledit résultat du CNN de segmentation (400) des structures du système nerveux étant amélioré par des réseaux antagonistes génératifs, GAN.

8. Procédé selon l'une quelconque des revendications précédentes, lesdites images de balayage médical reçues étant collectées à partir d'un scanner peropératoire.

9. Procédé selon l'une quelconque des revendications précédentes, lesdites images de balayage médical reçues étant collectées à partir d'un scanner stationnaire pré-chirurgical.

10. Système mis en œuvre par ordinateur comprenant :
- au moins un support de stockage non transitoire lisible par processeur (910) qui stocke au moins une instruction exécutable par processeur (915) ou donnée ; et
- au moins un processeur (920) couplé en communication à au moins un support de stockage non transitoire lisible par processeur (910), au moins un processeur (920) étant configuré pour exécuter les étapes du procédé selon l'une quelconque des revendications précédentes.
